# EUROPEAN PATENT APPLICATION

(11) **EP 4 628 057 A1**
(43) Date of publication of application: **08.10.2025**
(21) Application number: 24168034.7
(22) Date of filing: 02.04.2024
(51) Int. Cl.: A61F 13/15, A61F 13/531, D04H 1/425, D04H 1/44, D04H 1/732

(54) **SUPPORT-BELT-FREE COMPRESSION OF PARTICLE-FIBRE INTERMIXTURES WITH SINGLE MASTER BELT**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is a process for compressing intermixtures of fibres, such as cellulosic pulp fibers, with particles, such as superabsorbent particles.

## Description

### Field of the invention

The present invention is a process for compressing intermixtures of fibres, such as cellulosic pulp fibers, with particles, such as superabsorbent particles.

### Background

It is well known to compress fluff structures, such as used for tissues but also for absorbent products such as hygiene products such as diapers, bed pads, or wipes, be it with or without additives such as particulate additive, such as superabsorbent (SAP) particles.

Such structures are often produced by a dry forming or airlaid process, whereby the fluff or fluff and SAP mix is deposited in a laydown region on a moving foraminous surface, such as a foraminous transfer belt, which further transfers the fluff or fluff and SAP mix to a compression unit. In order to keep the fluff or fluff/SAP mix on the transfer belt, air is sucked through the structure and the belt. Optionally, carrier webs such as tissues or nonwoven webs, may be added prior to or after the lay-down.

For compression at lower pressures the fluff or fluff/SAP mix is often transferred together with the transfer belt through the nip of the compression unit. For higher compression pressions, the fluff or fluff/SAP mix is separated from the transfer belt, often after a first lower pressure compression step.

Such approaches are known from e.g., EP1032342 (McAirlaid's) describing the compaction of cellulose structures, optionally with superabsorbent particles, whereby in a first lower pressure compression the transport belt is guided through the nip, whilst for the second high pressure compression the pre-compacted web is guided without transport belt into the nip. Also, EP4119111A1 (Fameccanica) describes the compression of a mix of fibers and superabsorbent particulates in a compression unit, to which the mix is fed by a first conveyor belt and after which the compressed web is received by a second conveyor belt, without a conveyor belt running through the compression unit.

However, there remain several problems with such approaches, especially for fluff/SAP mixes with relatively high amounts of SAP.

When the transfer belt is fed together with the fluff/SAP mix, SAP is passing through foraminous transfer belt. This not only implies a loss of functional material, but also increases the burden for subsequent air filtering of the vacuum suction air. Further, fine particles as may be carried with the air, tend to block openings of the foraminous belt, such that the air flow resistance may be increased and higher energy may be required to achieve the required vacuum. Further, as compression pressure and/or SAP relative SAP content increase, the transfer belt may be damages, such as by an increased roughness or grounded openings.

When support belt is not running through compression, each compression unit requires an infeed and a receiving transfer system, such as foraminous belts. This not only requires multiple drive and control systems, but also carries the risk of mismatched transfer speeds, as may result in poor quality due to tearing or crinkles.

Henceforth it is an object of the present invention to avoid such problems.

### Summary

The present invention is a method for forming a compressed air-laid web comprising SAP and pulp. The method comprising the steps of
a) providing
   a1) a continuous loop foraminous belt system with a continuous foraminous belt and foraminous belt guide rolls,
      whereby the movement of the foraminous belt in a laydown region defines a machine direction MD from upstream towards downstream, further cross-machine CD, and a height direction,
   a2) at least one compression system;
   a3) a suction system comprising at least a first and a second suction box adapted to suck air through the foraminous belt;
b) optionally providing a carrier, preferably a cellulosic tissue, and positioning it onto the foraminous belt machine directionally before the laydown region;
c) providing SAP from an SAP supply system and pulp from a pulp supply system,
   c1) intermixing these, preferably within a forming box;
   c2) forming a pulp/SAP intermix by depositing these in the laydown region height directionally above the foraminous belt
      onto the carrier on the foraminous belt, if the carrier is present,
      or directly onto the foraminous belt,
      supported by a suction provided by a first suction box;
d) transferring the pulp/SAP intermix on the carrier, if present, towards, but not into, a first compression unit,
   thereby optionally compressing the pulp/SAP intermix on the carrier, if present, in a pre-compression unit together with the foraminous belt;
e) compressing the pulp/ SAP intermix and the carrier, if present, in a first compression unit separated from the foraminous belt by
   e1) transferring the pulp / SAP intermix and the carrier, if present, towards the first compression unit (1510), preferably aided by first suction box, but not into the nip of the first compression unit;
   e2) separating the pulp / SAP intermix and the carrier, if present, from the foraminous belt;
   e3) feeding the pulp/SAP intermix and the carrier, if present, to the nip of the first compressing unit and compressing the intermix and the carrier, if present, to form a compressed intermix;
   e3) concurrently guiding the foraminous belt around the compressing unit via first bypass guide rolls;
   e4) combining the compressed pulp / SAP intermix and the carrier, if present, and the foraminous belt;
f) optionally compressing the compressed pulp / SAP mix and the carrier, if present, in a second compression unit by
   f1) transferring the compressed pulp / SAP mix and the carrier, if present, towards the second compression unit, preferably via a second suction box, but not into the nip of the second compression unit;
   f2) separating the pulp / SAP intermix and the carrier, if present, from the foraminous belt;
   f3) feeding the pulp/SAP intermix and the carrier, if present, to the nip of the second compressing unit and compressing the pulp / SAP intermix and the carrier, if present, to form further compressed pulp / SAP intermix;
   f4) concurrently guiding the foraminous belt around the second compressing unit via second bypass guide rolls;
   f5) combining the further compressed pulp / SAP intermix and the carrier, if present, and the foraminous belt;
g) transferring the compressed pulp / SAP intermix and the carrier, if present, on the foraminous belt to a further process unit by
   g1) separating the compressed pulp /SAP intermix and the carrier, if present, and the foraminous belt;
   g2) feeding the compressed pulp / SAP intermix and the carrier, if present, to the further processing unit;
   g3) guiding the foraminous belt towards the laydown region via main guide rolls.

In the step c), the providing of the SAP may be executed at a level of more than about 10 %, preferably more than about 30 %, more preferably more than about 50 %, even more preferably more than about 60 %, or even more than about 70 %, all percentages based on the weight of SAP and pulp.

In an optional variant, in step e) the compressing may be executed with a smooth surface compression unit and in the step f) the compressing may be executed with a patterned surface compression unit.

### Brief description of the figures

Fig. 1 depicts an apparatus for executing the present invention.
Fig. 2 depicts a variant of an apparatus for executing the present invention.
The figures are schematically only, and not to scale. Same numerals refer to same or equivalent features or elements, single (`) or multiple (", "', ...) apostrophes indicate multiple features, such a left and right or front and back, etc.

### Detailed description

The continuous process according to the present invention may be very suitably applied in the manufacturing of absorbent structures, such as may be employed in absorbent articles such as diapers, feminine hygiene products, incontinence devices, but also absorbent pads such as bed pads or for food trays. An absorbent material can also be used as an absorbent core in any device used to absorb body exudates (e.g., urine, breast milk, blood, serum). In the process of the present invention short fibers, such as cellulosic fibers, and Superabsorbent polymer (SAP) particles are intensively intermixed, preferably at relatively high levels of SAP. The process according to the present invention may be used to produce such absorbent structures as such, or it may be suitably directly integrated in the manufacturing process for making the absorbent article.

An absorbent material of the present invention can be used directly by a manufacturer of the absorbent article without the need for any additional processing by that manufacturer other than cutting or folding to the desired size and shape for the absorbent article.

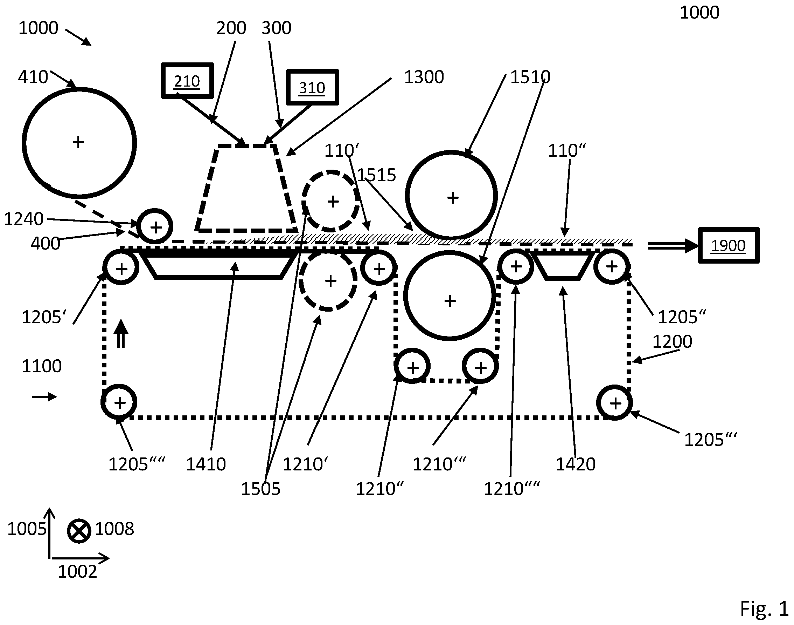

The principle of the present invention is now explained in more detail by referring to Fig. 1. This should, however not seen to imply limitation the present invention.

A process according to the present invention is operated on an apparatus 1000 for forming a web 100' by mixing SAP material 200 and short fibers 300.

The selection of SAP particles 200 is not particularly limited, and may be of in the form of particulate matter, flakes, fibers and the like. Particulate forms are preferred, including spherical or irregularly shaped granules, aggregates and agglomerates. Exemplary and preferred superabsorbent materials include salts of crosslinked polyacrylic acid such as sodium polyacrylate. superabsorbent materials are commercially available (e.g., BASF, Germany). Other suitable SAP materials may be based on crosslinked Carboxymethyl Cellulose, such as available from Evonik, Germany, or on crosslinked starch as from GreenThings, Germany.

Similarly, short fibers 300 may be selected from a broad range of materials and preferably are plant derived, such as cellulosic fibers including wood pulp, cotton, flax, and peat moss. The present invention also contemplates that short cut synthetic fibers (up to about 10 percent) may be incorporated in the absorbent core. Wood pulp is preferred. Pulps can be obtained from mechanical or chemi-mechanical, sulfite, kraft, pulping reject materials, organic solvent pulps, etc. Both softwood and hardwood species are useful, which may be chemically treated, e.g., with debonding or cross-linking agents. Softwood pulps are preferred.

The present invention is particularly suited to be applied to short fiber / SAP intermixes that comprise overall relatively high amounts of SAP, such as more than about 30 w-% of the combined weight of short fibers and SAP, or more than about 40 w-%, or more than about 50 w-%, or more than about 60 w-% or even more than about 70 w-%.

The short fibers 300 and the SAP 200 are intensely intermixed before being deposited as web 100' onto a foraminous belt 1200 as a collection device, preferably with a homogeneous distribution across its thickness direction 1005, though a z- and/or y-directional distribution profile can be executed. The intermixing is preferably made in a forming box 1300, which is typically oriented such that its lower portion along gravity is open towards the laydown region 1310 towards the foraminous belt 1000.

Depending on the intended use, the basis weight of the web may be more than about 20 gsm or more than about 50 gsm, or more than about 100 gsm or more than about 300 gsm, but typically less than about 2000 gsm, or less than about 1000 gsm.

In an option as also depicted in Fig. 1, a carrier web 400 may be provided by a carrier web supply 410 and fed via appropriate carrier web guide roll 1410 towards the laydown region 1310. It should be noted that the carrier web 400 has to be air permeable. In a particularly preferred execution, the carrier is a conventional paper tissue, preferably at less than about 30 gsm, or less than about 20 gsm, or less than about 15 gsm, or less than about 10 gsm.

The apparatus 1000 comprises a first vacuum creating suction box 1410 positioned at least underneath the laydown region 1310, by which the intermix of the short fibers and the SAP is positioned on the foraminous belt 1200, with the optional carrier 400 positioned between the intermixture and the foraminous belt 1200.

The direction of movement of said foraminous belt in said laydown section defines the machine direction (MD) 1002, with the width direction (CD) 1008 perpendicular thereto and a height or z-direction 1005 perpendicular to both.

By this deposition, an uncompressed short fiber / SAP intermix web 100' is formed, which is fairly lofty as being essentially only compacted by the vacuum suction of the first suction box 1410.

The foraminous belt 1200 is part of a foraminous belt system 1100 and is run in a continuous loop via sets of several guide rolls 1205, 1210, 1220. The first set of guide rolls, main path guide rolls 1205', ", "', "", covers the essential elements for looping the foraminous belt 1200. Whilst showing the four main path guide rolls 1205 in an essentially rectangular arrangement, there may be more than four main path guide rolls 1205, optionally of varying size, and optionally arranged non-rectangularly, such in a trapeze, arrangement.

The foraminous belt 1200 can be of conventional type, such as known for air-laying processes, commercially available from e.g., GKD Group - Kufferath AG, Germany, or Rai-Tilliers^{®}, France, being optimized for air permeability whilst preventing short fibers and minimizing particle pass-through.

In an optional approach, the web 100' may be pre-compressed in a pre-compressing unit 1505, wherein the web may be compressed whilst being positioned on the foraminous belt, as known from the prior art. However, in view of the above referenced draw-backs of this approach, the compression should only be executed at lower pressures and/ or low relative amounts of particles. The web 100' is then transferred towards a first compression unit 1510 on the foraminous belt, but not into the nip 1515, as the foraminous belt and the web 100' are separated just before the nip. This is in contrast to conventional systems, where the foraminous belt is carrying the web 100' into and through the nip of the pair of compression rolls 1510, or where a first foraminous belt system transfers the web to the compression unit and a second foraminous belt system receives the compressed web 100" thereafter.

Especially for webs with higher amount of SAP, the first approach, may result in SAP being pressed through the foraminous belt, which not only results in material loss, but also in an increased air cleaning resp. filtering requirement for the process air and the potential blockage of the opening of the foraminous belt may reduce the air permeability, requiring to increase the air suction via an increase of the vacuum, requiring higher energy consumption. Also, the foraminous belt may be damaged, with increased roughness and / or damage of the openings.

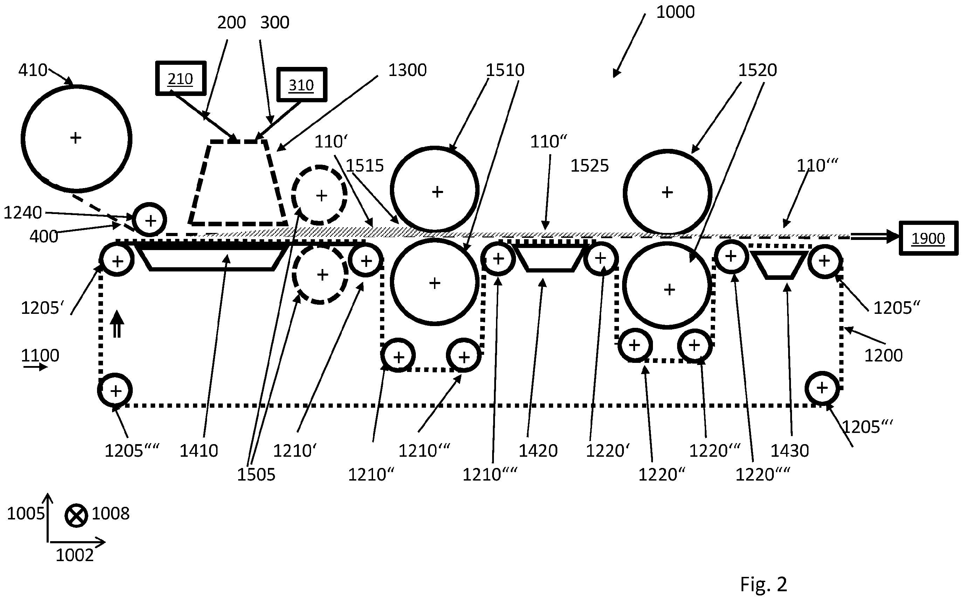

The second conventional approach of using a first and a second foraminous belt system requires duplicating the drive and control systems, especially to maintain matched speed of the two systems. Thus, according to the present invention, the foraminous belt 1200 and the uncompressed web 100' are separated just prior to the compression unit 1510. The web is compressed in the nip 1515, whilst the foraminous belt 1200 is guided by a first set of bypass guide rolls 1210 around the first compression unit 1510 so as to be rejoined with the now compressed web 100" after passing through the compression unit 1510. A second suction unit supports the contact between the compressed web 100" and the foraminous belt before the compressed web and the foraminous belt are separated again, with the web being transferred to a further downstream processing step and the foraminous belt 1200 being looped back to the laydown region 1310 via main path guide rolls 1205. If a carrier web 400 is employed, it is fed together with web 100' towards and through the nip 1515. Fig. 2 represents a variant of the process according to the present invention, wherein a second compression step is executed on a second compression unit 1520 downstream of the first. The forming of the web and the first compression are executed in analogy to the above. After the first compression unit 1510, the compressed web 100" is transferred on the foraminous belt 1200, preferably via a further vacuum suction box 1430 towards a second compression unit 1520. The foraminous belt 1200 and the compressed web 100" are separated just prior to the second compression unit 1520. The web is compressed in the second nip 1525, whilst the foraminous belt 1200 is guided by a second set of bypass guide rolls 1220 around the second compression unit 1520 so as to be rejoined with the now further compressed web 100‴ after passing through the second compression unit 1520. A further suction unit supports the contact between the compressed web 100‴ and the foraminous belt before the compressed web and the foraminous belt are separated again, with the web being transferred to a further downstream processing step and the foraminous belt 1200 being looped back to the laydown region 1310 via main path guide rolls 1205.

If a carrier web 400 is employed, it is fed together with web 100" towards and through the second nip 1525.

## Claims

1. A method for forming a compressed air-laid web (100) comprising SAP (200) and pulp (300), said method comprising the steps of
a) providing
a1) a continuous loop foraminous belt system (1000) with a continuous foraminous belt (1200) and foraminous belt guide rolls (1205, 1210, 1220),
whereby the movement of said foraminous belt in a laydown region (1310) defines a machine direction MD (1002) from upstream towards downstream, further cross-machine CD (1008), and a height direction (1005),
a2) at least one compression system (1510, 1520);
a3) a suction system (1400) comprising at least a first (1410) and a second (1420) suction box adapted to suck air through said foraminous belt (1200);
b) optionally providing a carrier (400), preferably a cellulosic tissue, and positioning it onto said foraminous belt (1200) machine directionally before said laydown region (1310);
c) providing SAP (200) from an SAP supply system (210) and pulp (300) from a pulp supply system (310),
c1) intermixing these, preferably within a forming box (1300);
c2) forming a pulp/SAP intermix (110') by depositing these in said laydown region (1310) height directionally above said foraminous belt (1200)
onto said carrier (400) on said foraminous belt (1200), if said carrier (400) is present, or directly onto said foraminous belt (1200),
supported by a suction provided by a first suction box (1410);
d) transferring said pulp/SAP intermix (110') on said carrier (400), if present, towards, but not into, a first compression unit (1510),
thereby optionally compressing said pulp/SAP intermix (110') on said carrier (400), if present, in a pre-compression unit (1505) together with said foraminous belt;
e) compressing said pulp/ SAP intermix (110') and said carrier (400), if present, in a first compression unit (1510) separated from said foraminous belt (1200) by
e1) transferring said pulp / SAP intermix (110) and said carrier (400), if present, towards said first compression unit (1510), preferably aided by first suction box (1410), but not into the nip (1515) of said first compression unit (1510);
e2) separating said pulp / SAP intermix (110') and said carrier (400), if present, from said foraminous belt (1200);
e3) feeding said pulp/SAP intermix (110') and said carrier (400), if present, to said nip (1515) of said first compressing unit (1510) and compressing said intermix (110') and said carrier (400), if present, to form a compressed intermix (110");
e3) concurrently guiding said foraminous belt (1200) around said compressing unit (1510) via first bypass guide rolls (1210);
e4) combining said compressed pulp / SAP intermix (110") and said carrier (400), if present, and said foraminous belt (1200);
f) optionally compressing said compressed pulp / SAP mix (110") and said carrier (400), if present, in a second compression unit (1520) by
f1) transferring said compressed pulp / SAP mix (110") and said carrier (400), if present, towards said second compression unit (1520), preferably via a second suction box (1420), but not into the nip (1525) of said second compression unit (1520);
f2) separating said pulp / SAP intermix (1210") and said carrier (400), if present, from said foraminous belt (1200);
f3) feeding said pulp/SAP intermix (110") and said carrier (400), if present, to the nip (1525) of said second compressing unit (1520) and compressing said pulp / SAP intermix (110") and said carrier (400), if present, to form further compressed pulp / SAP intermix (110‴);
f4) concurrently guiding said foraminous belt (1200) around said second compressing unit (1520) via second bypass guide rolls (1220);
f5) combining said further compressed pulp / SAP intermix (110'") and said carrier (400), if present, and said foraminous belt (1200);
g) transferring said compressed pulp / SAP intermix (100", 100‴) and said carrier (400), if present, on said foraminous belt (1200) to a further process unit (1900) by
g1) separating said compressed pulp /SAP intermix (100", 1100‴) and said carrier (400), if present, and said foraminous belt (1200);
g2) feeding said compressed pulp / SAP intermix (100", 100‴) and said carrier (400), if present, to said further processing unit (1900);
g3) guiding said foraminous belt (1200) towards said laydown region (1310) via main guide rolls (1205).

2. A method for forming a compressed air-laid web (100) according to claim 1, wherein
in said step c) the providing of the SAP is executed at a level of more than about 10 %, preferably more than about 30 %, more preferably more than about 50 %, even more preferably more than about 60 %, or even more than about 70 %, all percentages based on the weight of SAP and pulp.

3. A method for forming a compressed air-laid web (100) according to claim 1 or 2, wherein
in said step e) the compressing is executed with a smooth surface compression unit (1510) and in said step f) the compressing is executed with a patterned surface compression unit (1510).
